(19) Europäisches Patentamt
European Patent Office
Office européen des brevets

(11) Veröffentlichungsnummer: **0 314 038 A1**

(12) **EUROPÄISCHE PATENTANMELDUNG**

(21) Anmeldenummer: **88117660.6**

(22) Anmeldetag: **24.10.88**

(51) Int. Cl.⁴: **C07D 211/90, C07D 401/12, C07D 401/14, C07D 413/14, A61K 31/445**

(30) Priorität: **27.10.87 CH 4223/87**

(43) Veröffentlichungstag der Anmeldung: **03.05.89 Patentblatt 89/18**

(84) Benannte Vertragsstaaten: **ES GR**

(71) Anmelder: **Byk Gulden Lomberg Chemische Fabrik GmbH**
**Byk-Gulden-Strasse 2**
**D-7750 Konstanz(DE)**

(72) Erfinder: **Flockerzi, Dieter, Dr.**
**Ackerweg 26**
**D-7753 Allensbach(DE)**
Erfinder: **Ulrich, Wolf-Rüdiger, Dr.**
**Hebelstrasse 2**
**D-7750 Konstanz(DE)**
Erfinder: **Sanders, Karl, Dr.**
**Felchengang 23**
**D-7750 Konstanz(DE)**
Erfinder: **Beller, Klaus-Dieter**
**Franz-Moser-Strasse 5**
**D-7750 Konstanz 16(DE)**
Erfinder: **Eistetter, Klaus, Dr.**
**Säntisblick 7**
**D-7750 Konstanz 19(DE)**
Erfinder: **Eltze, Manfrid, Dr.**
**Schützenstrasse 20**
**D-7750 Konstanz(DE)**
Erfinder: **Boer, Rainer, Dr.**
**Löhrystrasse 4**
**D-7750 Konstanz(DE)**
Erfinder: **Kohl, Bernhard, Dr.**
**Heinrich v. Tettingenstrasse 35a**
**D-7750 Konstanz 19(DE)**
Erfinder: **Amschler, Hermann, Dr.**
**Hohenhewenstrasse 19**
**D-7760 Radolfzell(DE)**
Erfinder: **Klemm, Kurt, Prof. Dr.**
**Im Weinberg 2**
**D-7753 Allensbach(DE)**

(54) **Neue Pyrrolidine.**

(57) Neue Pyrrolidine der Formel I,

EP 0 314 038 A1

Xerox Copy Centre

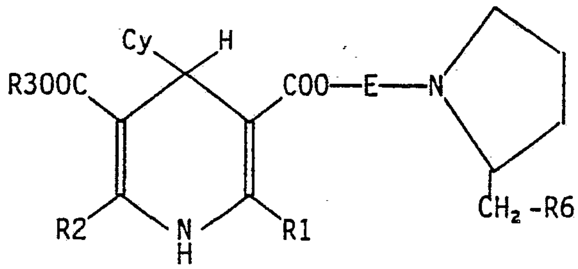

(I)

worin die Substituenten und Symbole die in der Beschreibung genannten Bedeutungen haben, sind neue Verbindungen mit überraschenden pharmakologischen Eigenschaften.

## Neue Pyrrolidine

Anwendungsgebiet der Erfindung

Die Erfindung betrifft neue Pyrrolidine, Verfahren zu ihrer Herstellung, ihre Verwendung und sie enthaltende Arzneimittel. Die erfindungsgemäßen Verbindungen werden in der pharmazeutischen Industrie zur Herstellung von Arzneimitteln eingesetzt.

Bekannter technischer Hintergrund

Es ist bekannt, daß bestimmte, auf verschiedene Weise substituierte 1,4-Dihydropyridinderivate pharmakologisch nützliche Eigenschaften aufweisen. Überraschenderweise wurde nun gefunden, daß die unten näher beschriebenen neuen Verbindungen besonders interessante pharmakologische Eigenschaften aufweisen, durch die sie sich von den Verbindungen des Standes der Technik in vorteilhafter Weise unterscheiden.

Beschreibung der Erfindung

Gegenstand der Erfindung sind neue Pyrrolidine der Formel I

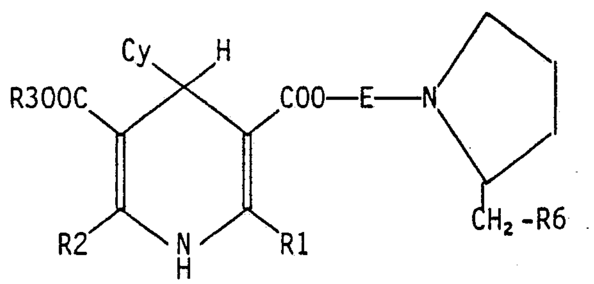

(I)

worin Cy einen Cyclus der Formel

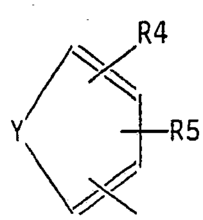

darstellt, in dem Y Sauerstoff (O), Schwefel (S), Vinylen (-CH = CH-), Azomethin (-CH = N-) oder eine Gruppe der Formel

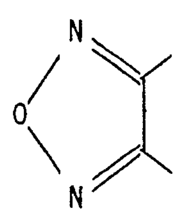

oder

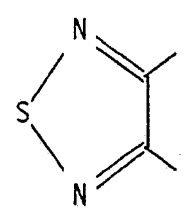

bedeutet,
R1 1-6C-Alkyl oder 3-7C-Alkoxyalkyl bedeutet,

R2 1-6C-Alkyl oder 3-7C-Alkoxyalkyl bedeutet,
R3 Wasserstoff, 1-6C-Alkyl oder 3-7C-Alkoxyalkyl bedeutet,
R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono- oder Di-1-4C-alkylamino bedeuten,
R6 Phenyl oder substituiertes Phenyl mit einem, zwei oder drei gleichen oder verschiedenen Substituenten aus der Gruppe Halogen, Hydroxy, Nitro, Cyano, Trifluormethyl, 1-4C-Alkyl, 1-4C-Alkoxy, 1-4C-Alkoxycarbonyl, 2-5C-Acyl, Amino oder Mono- oder Di-1-4C-alkylamino bedeutet und
E 2-5C-Alkylen bedeutet,
und die Salze dieser Verbindungen.

1-6C-Alkyl ist geradkettig oder verzweigt und bedeutet beispielsweise einen Hexyl-, Neopentyl-, Isopentyl-, Butyl-, i-Butyl-, sec.-Butyl-, t-Butyl-, Propyl-, Isopropyl- oder insbesondere Ethyl- oder Methylrest.

3-7C-Alkoxyalkyl steht beispielsweise für einen Ethoxyethyl-, Propoxyethyl-, Isopropoxyethyl-, Butoxyethyl-, Methoxypropyl-, 2-Methoxy-1-methylethyl-, 2-Ethoxy-1-methylethyl- oder insbesondere Methoxyethylrest.

Halogen im Sinne der Erfindung bedeutet Brom, Fluor und insbesondere Chlor.

1-4C-Alkyl ist geradkettig oder verzweigt und bedeutet beispielsweise einen Butyl-, i-Butyl-, sec.-Butyl-, t-Butyl-, Propyl-, Isopropyl-, Ethyl- oder insbesondere Methylrest.

1-4C-Alkoxy enthält neben dem Sauerstoffatom einen der vorstehend genannten 1-4C-Alkylreste. Bevorzugt sind der Methoxy- und der Ethoxyrest.

Ganz oder teilweise durch Fluor substituiertes 1-4C-Alkoxy ist beispielsweise 1,1,2,2-Tetrafluorethoxy, Trifluormethoxy, 2,2,2-Trifluorethoxy oder insbesondere Difluormethoxy.

1-4C-Alkoxycarbonyl enthält neben der Carbonylgruppe einen der vorstehend genannten 1-4C-Alkoxyreste. Bevorzugt sind der Methoxycarbonyl- und der Ethoxycarbonylrest.

2-5C-Acyl enthält neben der Carbonylgruppe einen der vorstehend genannten 1-4C-Alkylreste. Bevorzugt ist der Acetylrest.

Mono- oder Di-1-4C-alkylamino enthält neben dem Stickstoffatom einen oder zwei der vorstehend genannten 1-4C-Alkylreste. Bevorzugt ist Di-1-4C-alkylamino, und hier insbesondere Dimethyl-, Diethyl- oder Diisopropylamino.

2-5C-Alkylen ist geradkettig oder verzweigt und steht beispielsweise für Ethylen ($-CH_2-CH_2-$), Trimethylen ($-CH_2-CH_2-CH_2-$), Tetramethylen ($-CH_2-CH_2-CH_2-CH_2-$), Pentamethylen ($-CH_2-CH_2-CH_2-CH_2-CH_2-$), 1,2-Dimethylethylen, 1,1-Dimethylethylen, 2,2-Dimethylethylen, Isopropyliden, 1-Methylethylen und 2-Ethylpropylen, wobei Ethylen und Trimethylen bevorzugt sind.

Als Salze kommen alle Salze mit Säuren in Betracht. Besonders erwähnt seien die pharmakologisch verträglichen Salze der in der pharmazeutischen Industrie üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Metembonat, Stearat, Tosilat, 2-Hydroxy-3-naphthoat, 3-Hydroxy-2-naphthoat oder Mesilat, aber auch Salze mit Bumetanid, Furosemid, Azosemid, Galosemid, Besunid, Piretanid, Etacrynsäure, Tienilinsäure oder 4-Chlor-sulfamoyl-benzoesäure.

Hervorzuhebende erfindungsgemäße Verbindungen sind solche der Formel I, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl, 3-Trifluormethylphenyl oder Benzoxdiazolyl bedeutet,
R1 Methyl, Ethyl oder Methoxyethyl bedeutet,
R2 Methyl, Ethyl oder Methoxyethyl bedeutet,
R3 Methyl, Ethyl oder Methoxyethyl bedeutet,
R6 Phenyl, 2-Chlorphenyl, 3-Chlorphenyl, 4-Chlorphenyl, 2,3-Dichlorphenyl, 3,4-Dichlorphenyl, -2,4-Dichlorphenyl, 4-Bromphenyl, 4-Fluorphenyl, 3-Trifluormethylphenyl, 2-Methoxyphenyl, 3-Methoxyphenyl, 4-Methoxyphenyl, 3,4-Dimethoxyphenyl, 3,4,5-Trimethoxyphenyl, 2-Methylphenyl, 3-Methylphenyl, 3-Chlor-4-methylphenyl, 3,4-Dimethylphenyl, 4-Aminophenyl, 2,4-Diaminophenyl, 4-Nitrophenyl, 3-Hydroxyphenyl, 4-Hydroxyphenyl, 3,4-Dihydroxyphenyl oder 3,4,5-Trihydroxyphenyl bedeutet und

E Ethylen ($-CH_2CH_2-$), Trimethylen ($-CH_2-CH_2-CH_2-$), Tetramethylen ($-CH_2-CH_2-CH_2-CH_2-$) oder Pentamethylen ($-CH_2-CH_2-CH_2-CH_2-CH_2-$) bedeutet,
und die Salze dieser Verbindungen.

Besonders hervozuhebende erfindungsgemäße Verbindungen sind solche der Formel I, worin
Cy 3-Nitrophenyl, 2-Chlorphenyl, 2,3-Dichlorphenyl oder 2-Trifluormethylphenyl bedeutet,
R1 Methyl oder Ethyl bedeutet,
R2 Methyl oder Ethyl bedeutet,
R3 Methyl oder Ethyl bedeutet,
R6 4-Chlorphenyl, 4-Methoxyphenyl, 3,4-Dimethoxyphenyl oder 4-Nitrophenyl und
E Ethylen ($-CH_2CH_2-$) oder Trimethylen ($-CH_2-CH_2-CH_2-$) bedeutet,
und die Salze dieser Verbindungen.

Bevorzugte erfindungsgemäße Verbindungen sind solche der Formel I, worin
Cy 3-Nitrophenyl oder 2,3-Dichlorphenyl bedeutet,
R1 Methyl oder Ethyl bedeutet,
R2 Methyl oder Ethyl bedeutet,
R3 Methyl oder Ethyl bedeutet,
R6 4-Chlorphenyl oder 4-Nitrophenyl und
E Ethylen ($-CH_2CH_2-$) oder Trimethylen ($-CH_2-CH_2-CH_2-$) bedeutet,
und ihre Salze.

Besonders bevorzugte erfindungsgemäße Verbindungen sind solche der Formel I, worin
Cy 3-Nitrophenyl bedeutet,
R1 Methyl bedeutet,
R2 Methyl bedeutet,
R3 Methyl oder Ethyl bedeutet,
R6 4-Chlorphenyl und
E Ethylen ($-CH_2CH_2-$) oder Trimethylen ($-CH_2-CH_2-CH_2-$) bedeutet,
und ihre Salze.

Die Verbindungen der Formel I besitzen an der 4-Position im 1,4-Dihydropyridin und an der 2-Position im Pyrrolidinring je ein Chiralitätszentrum. Die Erfindung umfaßt daher sowohl die Enantiomeren als auch die Diastereomeren, sowie deren Gemische und Racemate. Besonders bevorzugt sind in diesem Zusammenhang die Diastereomeren, die in der 4-Position im Dihdyropyridin die gleiche Konfiguration aufweisen, wie das als Ausgangsverbindung eingesetzte (-)-1-Ethoxymethyl-1,4-dihydro-5-methoxycarbonyl-2,6-dimethyl-4-(3-nitrophenyl)-pyridin-3-carbonsäure-Cinchonidinsalz, das das linear polarisierte Licht der Wellenlänge 589 nm mit $[\alpha]_D^{22} = + 63{,}4^\circ$ (c = 1, Chloroform) dreht; ferner sind die Diastereomeren bevorzugt, die in der 2-Position des Pyrrolidinringes die gleiche Konfiguration aufweisen, wie das als Ausgangsverbindung eingesetzte (-)-2-(4-Chlorbenzyl)-pyrrolidin-hydrochlorid, das das linear polarisierte Licht der Wellenlänge 589 mm mit $[\alpha]_D^{22} = - 31{,}7^\circ$ (c = 1, Methanol) dreht.

Als bevorzugte erfindungsgemäße Verbindungen seien beispielsweise genannt:
1,4-Dihydro-2,6-dimethyl-4-(+)-(3-nitrophenyl)-pyridin-3,5-dicarbonsäure-3-methyl-5-{3-[2-(-)-(4-chlorbenzyl)-pyrrolidinyl-1]-propyl}-ester
1,4-Dihydro-2,6-dimethyl-4-(+)-(3-nitrophenyl)-pyridin-3,5-dicarbonsäure-3-ethyl-5-{3-[2-(-)-(4-chlorbenzyl)-pyrrolidinyl-1]-propyl}-ester
1,4-Dihydro-2,6-dimethyl-4-(+)-(3-nitrophenyl)-pyridin-3,5-dicarbonsäure-3-(2-methoxyethyl-5-{3-[2-(-)-(4-chlorbenzyl)-pyrrolidinyl-1]-propyl}-ester
1,4-Dihydro-2,6-dimethyl-4-(+)-(3-nitrophenyl)-pyridin-3,5-dicarbonsäure-3-propyl-2)-5-{3-[2-(-)-(4-chlorbenzyl)-pyrrolidinyl-1]-propyl}-ester
1,4-Dihydro-2,6-dimethyl-4-(+)-(3-nitrophenyl)-pyridin-3,5-dicarbonsäure-3-methyl-5-{2-[2-(-)-(4-chlorbenzyl)-pyrrolidinyl-1]-ethyl}-ester
1,4-Dihydro-2,6-dimethyl-4-(+)-(3-nitrophenyl)-pyridin-3,5-dicarbonsäure-3-ethyl-5-{2-[2-(-)-(4-chlorbenzyl)-pyrrolidinyl-1]-ethyl}-ester
1,4-Dihydro-2,6-dimethyl-4-(+)-(3-nitrophenyl)-pyridin-3,5-dicarbonsäure-3-(2-methoxyethyl)-5-{2-[2-(-)-(4-chlorbenzyl)-pyrrolidinyl-1]-ethyl}-ester
1,4-Dihydro-2,6-dimethyl-4-(+)-(3-nitrophenyl)-pyridin-3,5-dicarbonsäure-3-(propyl-2)-5-{2-[2-(-)-(4-chlorbenzyl)-pyrrolidinyl-1]-ethyl}-ester
4-(+)-(2,3-Dichlorphenyl)-1,4-dihydro-2,6-dimethyl-pyridin-3,5-dicarbonsäure-3-methyl-5-{3-[2-(-)-(4-chlorbenzyl)-pyrrolidinyl-1]-propyl}-ester
4-(+)-(2,3-Dichlorphenyl)-1,4-dihydro-2,6-dimethyl-pyridin-3,5-dicarbonsäure-3-ethyl-5-{3-[2-(-)-(4-chlorbenzyl)-pyrrolidinyl-1]-propyl}-ester

4-(+)-(2,3-Dichlorphenyl)-1,4-dihydro-2,6-dimethyl-pyridin-3,5-dicarbonsäure-3-(2-methoxyethyl)-5-{3-[2-(-)-(4-chlorbenzyl)-pyrrolidinyl-1]-propyl}-ester

4-(+)-(2,3-Dichlorphenyl)-1,4-dihydro-2,6-dimethyl-pyridin-3,5-dicarbonsäure-3-(propyl-2)-5-{3-[2-(-)-(4-chlorbenzyl)-pyrrolidinyl-1]-propyl}-ester

4-(+)-(2,3-Dichlorphenyl)-1,4-dihydro-2,6-dimethyl-pyridin-3,5-dicarbonsäure-3-methyl-5-{2-[2-(-)-(4-chlorbenzyl)-pyrrolidinyl-1]-ethyl}-ester

4-(+)-(2,3-Dichlorphenyl)-1,4-dihydro-2,6-dimethyl-pyridin-3,5-dicarbonsäure-3-ethyl-5-{2-[2-(-)-(4-chlorbenzyl)-pyrrolidinyl-1]-ethyl}-ester

4-(+)-(2,3-Dichlorphenyl)-1,4-dihydro-2,6-dimethyl-pyridin-3,5-dicarbonsäure-3-(2-methoxyethyl)-5-{2-[2-(-)-(4-chlorbenzyl)-pyrrolidinyl-1]-propyl}-ester

4-(+)-(2,3-Dichlorphenyl)-1,4-dihydro-2,6-dimethyl-pyridin-3,5-dicarbonsäure-3-(propyl-2)-5-{2-[2-(-)-(4-chlorbenzyl)-pyrrolidinyl-1]-ethyl}-ester

4-(+)-(2,1,3-Benzoxdiazol-4-yl)-1,4-dihydro-2,6-dimethyl-pyridin-3,5-dicarbonsäure-3-methyl-5-{3-[2-(-)-(4-chlorbenzyl)-pyrrolidinyl-1]-propyl}-ester

4-(+)-(2,1,3-Benzoxdiazol-4-yl)-1,4-dihydro-2,6-dimethyl-pyridin-3,5-dicarbonsäure-3-methyl-5-{2-[2-(-)-(4-chlorbenzyl)-pyrrolidinyl-1]-ethyl}-ester

1,4-Dihydro-2,6-dimethyl-4-(+)-(3-nitrophenyl)-pyridin-3,5-dicarbonsäure-3-methyl-5-{3-[2-(+)-(4-chlorbenzyl)-pyrrolidinyl-1]-propyl}-ester

1,4-Dihydro-2,6-dimethyl-4-(+)-(3-nitrophenyl)-pyridin-3,5-dicarbonsäure-3-ethyl-5-{3-[2-(+)-(4-chlorbenzyl)-pyrrolidinyl-1]-propyl}-ester

1,4-Dihydro-2,6-dimethyl-4-(+)-(3-nitrophenyl)-pyridin-3,5-dicarbonsäure-3-(2-methoxyethyl-5-{3-[2-(+)-(4-chlorbenzyl)-pyrrolidinyl-1]-propyl}-ester

1,4-Dihydro-2,6-dimethyl-4-(+)-(3-nitrophenyl)-pyridin-3,5-dicarbonsäure-3-(propyl-2)-5-{3-[2-(+)-(4-chlorbenzyl)-pyrrolidinyl-1]-propyl}-ester

1,4-Dihydro-2,6-dimethyl-4-(+)-(3-nitrophenyl)-pyridin-3,5-dicarbonsäure-3-methyl-5-{2-[2-(+)-(4-chlorbenzyl)-pyrrolidinyl-1]-ethyl}-ester

1,4-Dihydro-2,6-dimethyl-4-(+)-(3-nitrophenyl)-pyridin-3,5-dicarbonsäure-3-ethyl-5-{2-[2-(+)-(4-chlorbenzyl)-pyrrolidinyl-1]-ethyl}-ester

1,4-Dihydro-2,6-dimethyl-4-(+)-(3-nitrophenyl)-pyridin-3,5-dicarbonsäure-3-(2-methoxyethyl-5-{2-[2-(+)-(4-chlorbenzyl)-pyrrolidinyl-1]-ethyl}-ester

1,4-Dihydro-2,6-dimethyl-4-(+)-(3-nitrophenyl)-pyridin-3,5-dicarbonsäure-3-(propyl-2)-5-{2-[2-(+)-(4-chlorbenzyl)-pyrrolidinyl-1]-ethyl}-ester

4-(+)-(2,3-Dichlorphenyl)-1,4-dihydro-2,6-dimethyl-pyridin-3,5-dicarbonsäure-3-methyl-5-{3-[2-(+)-(4-chlorbenzyl)-pyrrolidinyl-1]-propyl}-ester

4-(+)-(2,3-Dichlorphenyl)-1,4-dihydro-2,6-dimethyl-pyridin-3,5-dicarbonsäure-3-ethyl-5-{3-[2-(+)-(4-chlorbenzyl)-pyrrolidinyl-1]-propyl}-ester

4-(+)-(2,3-Dichlorphenyl)-1,4-dihydro-2,6-dimethyl-pyridin-3,5-dicarbonsäure-3-(2-methoxyethyl-5-{3-[2-(+)-(4-chlorbenzyl)-pyrrolidinyl-1]-propyl}-ester

4-(+)-(2,3-Dichlorphenyl)-1,4-dihydro-2,6-dimethyl-pyridin-3,5-dicarbonsäure-3-propyl-2)-5-{3-[2-(+)-(4-chlorbenzyl)-pyrrolidinyl-1]-propyl}-ester

4-(+)-(2,3-Dichlorphenyl)-1,4-dihydro-2,6-dimethyl-pyridin-3,5-dicarbonsäure-3-methyl-5-{2-[2-(+)-(4-chlorbenzyl)-pyrrolidinyl-1]-ethyl}-ester

4-(+)-(2,3-Dichlorphenyl)-1,4-dihydro-2,6-dimethyl-pyridin-3,5-dicarbonsäure-3-ethyl-5-{2-[2-(+)-(4-chlorbenzyl)-pyrrolidinyl-1]-ethyl}-ester

4-(+)-(2,3-Dichlorphenyl)-1,4-dihydro-2,6-dimethyl-pyridin-3,5-dicarbonsäure-3-(2-methoxyethyl-5-{2-[2-(+)-(4-chlorbenzyl)-pyrrolidinyl-1]-ethyl}-ester

4-(+)-(2,3-Dichlorphenyl)-1,4-dihydro-2,6-dimethyl-pyridin-3,5-dicarbonsäure-3-(propyl-2)-5-{2-[2-(+)-(4-chlorbenzyl)-pyrrolidinyl-1]-ethyl}-ester

4-(+)-(2,1,3-Benzoxdiazol-4-yl)-1,4-dihydro-2,6-dimethyl-pyridin-3,5-dicarbonsäure-3-methyl-5-{3-[2-(+)-(4-chlorbenzyl)-pyrrolidinyl-1]-propyl}-ester

4-(+)-(2,1,3-Benzoxdiazol-4-yl)-1,4-dihydro-2,6-dimethyl-pyridin-3,5-dicarbonsäure-3-methyl-5-{2-[2-(+)-(4-chlorbenzyl)-pyrrolidinyl-1]-ethyl}-ester

1,4-Dihydro-2,6-dimethyl-4-(+)-(3-nitrophenyl)-pyridin-3,5-dicarbonsäure-3-methyl-5-{3-[2-(±)-(4-nitrobenzyl)-pyrrolidinyl-1]-propyl}-ester

1,4-Dihydro-2,6-dimethyl-4-(+)-(3-nitrophenyl)-pyridin-3,5-dicarbonsäure-3-ethyl-5-{3-[2-(±)-(4-nitrobenzyl)-pyrrolidinyl-1]-propyl}-ester

1,4-Dihydro-2,6-dimethyl-4-(+)-(3-nitrophenyl)-pyridin-3,5-dicarbonsäure-3-(2-methoxyethyl-5-{3-[2-(±)-(4-nitrobenzyl)-pyrrolidinyl-1]-propyl}-ester

1,4-Dihydro-2,6-dimethyl-4-( + )-(3-nitrophenyl)-pyridin-3,5-dicarbonsäure-3-(propyl-2)-5-{3-[2-(±)-(4-nitrobenzyl)-pyrrolidinyl-1]-propyl}-ester
1,4-Dihydro-2,6-dimethyl-4-( + )-(3-nitrophenyl)-pyridin-3,5-dicarbonsäure-3-methyl-5-{2-[2-(±)-(4-nitrobenzyl)-pyrrolidinyl-1]-ethyl}-ester
1,4-Dihydro-2,6-dimethyl-4-( + )-(3-nitrophenyl)-pyridin-3,5-dicarbonsäure-3-ethyl-5-{2-[2-(±)-(4-nitrobenzyl)-pyrrolidinyl-1]-ethyl}-ester
1,4-Dihydro-2,6-dimethyl-4-( + )-(3-nitrophenyl)-pyridin-3,5-dicarbonsäure-3-(2-methoxyethyl-5-{2-[2-(±)-(4-nitrobenzyl)-pyrrolidinyl-1]-ethyl}-ester
1,4-Dihydro-2,6-dimethyl-4-( + )-(3-nitrophenyl)-pyridin-3,5-dicarbonsäure-3-(propyl-2)-5-{2-[2-(±)-(4-nitrobenzyl)-pyrrolidinyl-1]-ethyl}-ester
4-( + )-(2,3-Dichlorphenyl)-1,4-dihydro-2,6-dimethyl-pyridin-3,5-dicarbonsäure-3-methyl-5-{3-[2-(±)-(4-nitrobenzyl)-pyrrolidinyl-1]-propyl}-ester
4-( + )-(2,3-Dichlorphenyl)-1,4-dihydro-2,6-dimethyl-pyridin-3,5-dicarbonsäure-3-ethyl-5-{3-[2-(±)-(4-nitrobenzyl)-pyrrolidinyl-1]-propyl}-ester
4-( + )-(2,3-Dichlorphenyl)-1,4-dihydro-2,6-dimethyl-pyridin-3,5-dicarbonsäure-3-(2-methoxyethyl-5-{3-[2-(±)-(4-nitrobenzyl)-pyrrolidinyl-1]-propyl}-ester
4-( + )-(2,3-Dichlorphenyl)-1,4-dihydro-2,6-dimethyl-pyridin-3,5-dicarbonsäure-3-propyl-2)-5-{3-[2-(±)-(4-nitrobenzyl)-pyrrolidinyl-1]-propyl}-ester
4-( + )-(2,3-Dichlorphenyl)-1,4-dihydro-2,6-dimethyl-pyridin-3,5-dicarbonsäure-3-methyl-5-{2-[2-(±)-(4-nitrobenzyl)-pyrrolidinyl-1]-ethyl}-ester
4-( + )-(2,3-Dichlorphenyl)-1,4-dihydro-2,6-dimethyl-pyridin-3,5-dicarbonsäure-3-ethyl-5-{2-[2-(±)-(4-nitrobenzyl)-pyrrolidinyl-1]-ethyl}-ester
4-( + )-(2,3-Dichlorphenyl)-1,4-dihydro-2,6-dimethyl-pyridin-3,5-dicarbonsäure-3-(2-methoxyethyl-5-{2-[2-(±)-(4-nitrobenzyl)-pyrrolidinyl-1]-ethyl}-ester
4-( + )-(2,3-Dichlorphenyl)-1,4-dihydro-2,6-dimethyl-pyridin-3,5-dicarbonsäure-3-(propyl-2)-5-{2-[2-(±)-(4-nitrobenzyl)-pyrrolidinyl-1]-ethyl}-ester
4-( + )-(2,1,3-Benzoxdiazol-4-yl)-1,4-dihydro-2,6-dimethyl-pyridin-3,5-dicarbonsäure-3-methyl-5-{3-[2-(±)-(4-nitrobenzyl)-pyrrolidinyl-1]-propyl}-ester
4-( + )-(2,1,3-Benzoxdiazol-4-yl)-1,4-dihydro-2,6-dimethyl-pyridin-3,5-dicarbonsäure-3-methyl-5-{2-[2-(±)-(4-nitrobenzyl)-pyrrolidinyl-1]-ethyl}-ester
1,4-Dihydro-2,6-dimethyl-4-( + )-(3-nitrophenyl)-pyridin-3,5-dicarbonsäure-3-methyl-5-{3-[2-( + )-(4-nitrobenzyl)-pyrrolidinyl-1]-propyl}-ester
1,4-Dihydro-2,6-dimethyl-4-( + )-(3-nitrophenyl)-pyridin-3,5-dicarbonsäure-3-methyl-5-{3-[2-(-)-(4-nitrobenzyl)-pyrrolidinyl-1]-propyl}-ester

und die Salze dieser Verbindungen.

Ein weiterer Gegenstand der Erfindung ist ein Verfahren zur Herstellung der erfindungsgemäßen Verbindungen und ihrer Salze. Das Verfahren ist dadurch gekennzeichnet, daß man

a) 1,4- Dihydropyridinderivate der Formel II

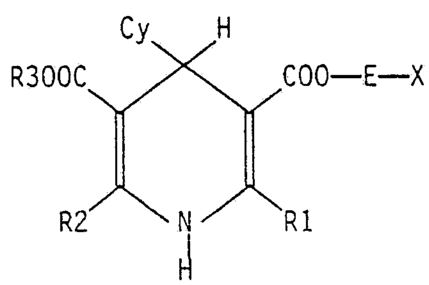

(II),

mit Aminen der Formel III

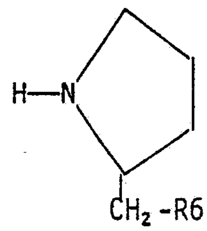

(III),

oder

b) Enamine der Formel IV

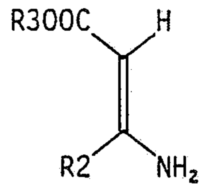

(IV),

mit Benzylidencarbonsäurederivaten der Formel V

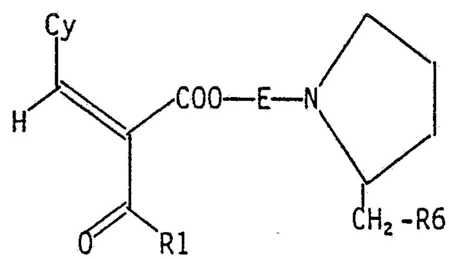

(V),

oder

c) Enaminderivate der Formel VI

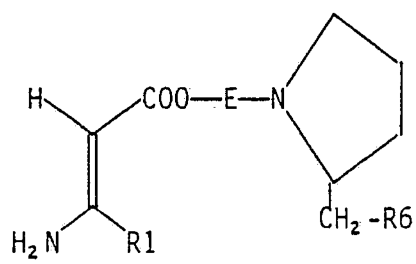

(VI),

mit Zimtsäurederivaten der Formel VII

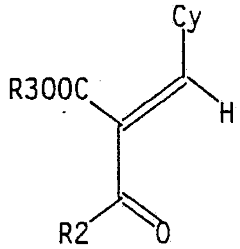

(VII),

als solche(n) oder in Form ihrer Salze umsetzt und gewünschtenfalls anschliessend erhaltene Salze in die freien Basen oder erhaltene Basen in die Salze überführt, wobei Cy, R1, R2, R3, R4, R5, R6 und E die oben angegebenen Bedeutungen haben und X eine Fluchtgruppe darstellt.

Ausgestaltungen des Verfahrens sind solche, bei denen in den Formeln II bis VII die Substituenten bzw. Symbole Cy, R1, R2, R3, R4, R5, R6 und E die in den Unter- und Nebenansprüchen angegebenen Bedeutungen haben und X eine Fluchtgruppe darstellt.

Die Umsetzung von II mit III erfolgt in einer Weise, wie sie für die Herstellung von tertiären Aminen bekannt ist. Je nach Art der Fluchtgruppe X, die vorzugsweise ein Halogenatom, insbesondere ein Chlor- oder Bromatom ist, kann die Reaktion gewünschtenfalls in Gegenwart einer Base (z.B. eines anorganischen Carbonates, wie Kaliumcarbonat) oder unter Einsetzung eines Überschusses von Amin III durchgeführt werden.

Die Umsetzung von IV mit V bzw. von VI mit VII erfolgt auf eine dem Fachmann bekannte Weise, beispielsweise so wie es in der Europäischen Patentanmeldung 176 956 beschrieben ist.

Die Umsetzung von II mit III, von IV mit V und von VI mit VII wird in geeigneten, vorzugsweise inerten organischen Lösungsmitteln durchgeführt. Beispielsweise seien genannt Kohlenwasserstoffe, wie Toluol oder Xylol; Ether, wie Dioxan, Diethylether, Tetrahydrofuran, Glykolmonoethylether oder Glykoldimethylether; oder Ketone, wie Aceton oder Ethylmethylketon; chlorierte Kohlenwasserstoffe, wie Methylenchlorid, Chloroform, Tetrachlorethylen oder Dichlorethan; oder andere organische Lösungsmittel, wie Dimethylformamid.

Die Reaktionstemperaturen können - je nach Reaktivität der Edukte - in einem weiten Bereich variieren. Im allgemeinen wird die Umsetzung bei Temperaturen zwischen 20 °C und 150 °C, vorzugsweise zwischen 20 °C und 100 °C, insbesondere bei der Siedetemperatur des verwendeten Lösungsmittels durchgeführt.

Die diastereomer reinen Verbindungen der Formel I und ihre Salze, die bevorzugter Gegenstand der Erfindung sind, erhält man einerseits, ausgehend von racemischen Gemischen der Verbindungen II, indem man diese mit einem enantiomer reinen Amin III umsetzt und die erhaltenen Diastereomeren auf übliche Weise trennt.

Alternativ erhält man die diastereomer reinen erfindungsgemäßen Verbindungen, indem man von racemischen Aminen III und enantiomer reinen 1,4-Dihydropyridinderivaten der Formel II ausgeht und anschließend eine Diastereomerentrennung durchführt, oder indem man von enantiomer reinen Aminen III und enantiomer reinen 1,4-Dihydropyridinderivaten der Formel II ausgeht.

Weiterhin erhält man die diastereomer reinen erfindungsgemäßen Verbindungen gemäß Verfahrensvarianten b oder c, indem man von enantiomer reinen Benzylidencarbonsäurederivaten V bzw. Enaminderivaten VI ausgeht. Nach Umsetzung von enantiomer reinem V bzw. VI mit einem Enamin IV bzw. Zimtsäurederivat VII erhält man ein Diastereomerengemisch, das auf übliche Weise getrennt werden kann.

Enantiomer reine 1,4-Dihydropyridinderivate der Formel II erhält man ausgehend von bekannten oder auf bekanntem Weg in analoger Weise herstellbaren enantiomer reinen N-geschützten 1,4-Dihydropyridincarbonsäure [Chem. Pharm. Bull. 28, 2809 (1980)] durch Umsetzung mit bifunktionellen Alkylenderivaten X-E-X, worin E die oben angebenen Bedeutungen hat und X eine Fluchtgruppe, insbesondere ein Halogenatom, bevorzugt ein Bromatom darstellt, und anschließende Abspaltung der N-Schutzgruppe.

Die Umsetzung der N-geschützten Dihydropyridincarbonsäuren, beispielsweise mit Dibromalkylenderivaten Br-E-Br, erfolgt bevorzugt unter basischen Bedingungen in Gegenwart eines Phasentransferkatalysators. Als Katalysatoren seien neben Oniumsalzen, wie z.B. Tetrabutylammoniumbromid oder Benzyltriethylammoniumchlorid, vor allem Kronenether, wie Dibenzo-[18]krone-6, Dicyclohexyl-[18]krone-6 und insbesondere [18]Krone-6 erwähnt.

Als eingesetzte Basen, die wenigstens im molaren Verhältnis, vorzugsweise im Überschuß eingesetzt werden, kommen anorganische Basen, wie Alkalimetallhydroxide (z.B. Natrium- oder Kaliumhydroxid), oder insbesondere Alkalimetallcarbonate (z.B. Natrium- oder vorzugsweise Kaliumcarbonat) in Frage. Beim Arbeiten in einem wasserfreien Lösungsmittel werden die verwendeten Hydroxide bzw. Carbonate vorzugsweise in feingepulverter Form eingesetzt.

Die Umsetzung erfolgt (je nach Art des Phasentransferkatalysators und der eingesetzten Base) in wasserhaltigen oder wasserfreien organischen Lösungsmitteln, oder in einem Gemisch aus Wasser und einem mit Wasser nicht oder kaum mischbaren organischen Lösungsmittel. Als Wasser/Lösungsmittelmischungen seien beispielsweise die Mischungen von Wasser mit Chloroform, Dichlormethan oder Benzol genannt. Als wasserhaltige oder wasserfreie Lösungsmittel seien beispielsweise Dichlormethan, Acetonitril oder Aceton genannt.

Die Wahl der Reaktionstemperatur hängt von den übrigen Reaktionsbedingungen ab, wobei in der Regel Temperaturen zwischen 20 °C und der Siedetemperatur des eingesetzten Lösungsmittels bevorzugt sind.

Enantiomer reine Amine III erhält man aus ihren Racematen durch Umsetzung mit enantiomer reinen optisch aktiven Säuren, wie beispielsweise die Di-0,0′-p-toluoylweinsäure oder die Di-0,0′-benzoylweinsäure, und anschließende Trennung der diastereomeren Salze auf üblichen Weg, z. B. durch Umkristallisation.

Die mit Hilfe dieser Methoden getrennten, konfigurativ einheitlichen diastereomeren Salze werden durch Zugabe vorzugsweise anorganischer Basen, wie z.B. Ammoniak, oder mit Hilfe von basischen Ionenaustauschern in die optisch aktiven, enantiomer reinen erfindungsgemäßen Verbindungen überführt.

Die Isolierung und Reinigung der erhaltenen erfindungsgemäßen Substanzen erfolgt in an sich bekannter Weise z. B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßriger Ammoniaklösung, in die freien Basen umgewandelt werden, welche wiederum in Säureadditionssalze überfeführt werden können. Auf diese Weise lassen sich pharmakologisch nicht verträgliche Säureadditionssalze in pharmakologisch verträgliche Säureadditionssalze umwandeln.

Die Ausgangsverbindungen II, III, IV, V, VI und VII sind literaturbekannt oder sie können in Analogie zu literaturbekannten Methoden hergestellt werden.

Die als Racemat eingesetzten Ausgangsverbindungen II können in an sich bekannter Weise z.B. nach folgendem Reaktionschema hergestellt werden:

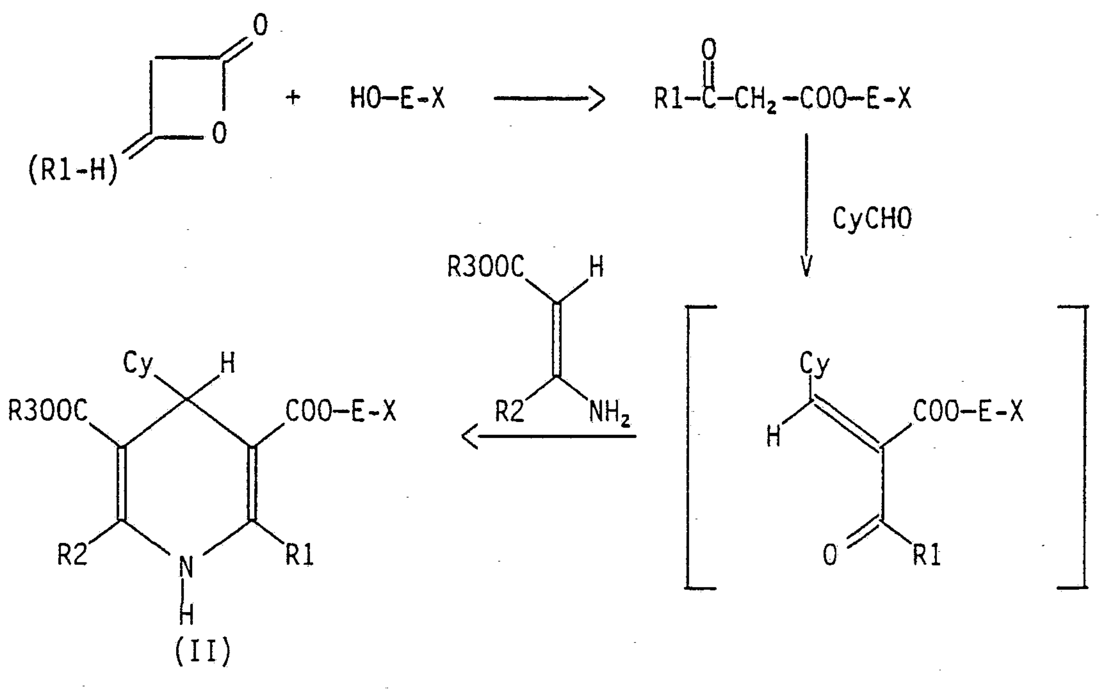


Die Amine der Formel III sind z. B. aus dem US-Patent 4,279,918 bekannt.

Die Benzylidencarbonsäurederivate V und die Zimtsäurederivate VII können beispielsweise in Analogie zu G. Jones ["The Knoevenagel Condensation" in Org. Reactions, Vol. XV, 204f (1967)] hergestellt werden. Die Enamine IV und die Enaminderivate VI sind beispielsweise analog A.C. Cope [J. Amer. Chem. Soc. 67, 1017 (1945)] erhältlich.

Die vorstehenden Herstellungsverfahren sind lediglich zur Verdeutlichung angegeben, und die Herstellung der erfindungsgemäßen Verbindungen der Formel I ist nicht auf diese Verfahren beschränkt. Vielmehr ist auch jede Modifikation dieser Verfahren in gleicher Weise für die Herstellung der erfindungsgemäßen Verbindungen anwendbar. Beispielsweise können die Racemate der erfindungsgemäßen Verbindungen auch nach jedem der in der internationalen Patentanmeldung WO 86/03748 erwähnten Verfahren aus entsprechenden Ausgangsverbindungen hergestellt werden.

Die folgenden Herstellungsbeispiele sollen die Erfindung näher erläutern, ohne sie einzuschränken. Schmp. bedeutet Schmelzpunkt, h steht für Stunden, Kp. steht für Siedepunkt, Zers. bedeutet Zersetzung.

## Beispiele

### Endprodukte

1. 1,4-Dihydro-2,6-dimethyl-4-(±)-(3-nitrophenyl)-pyridin-3,5-dicarbonsäure-3-ethyl-5-{3-[2-(±)-(4-chlorbenzyl)-pyrrolidinyl-1]-propyl}-ester-hydrochlorid

3,30 g (±)-2-Acetyl-3-(3-nitrophenyl)-acrylsäure-{3-[2-(4-chlorbenzyl)-pyrrolidinyl-1]-propyl}-ester und 0,91 g 3-Aminocrotonsäure-ethylester werden gemäß Verfahrensvariante b) in 80 ml 2-Propanol und 0,46 g Essigsäure 4 h unter Rückfluß zum Sieden erhitzt. Die erkaltete Lösung wird eingeengt und der Rückstand mit Wasser/Dichlormethan ausgeschüttelt. Nach dem Einengen der über Natriumsulfat getrockneten organischen Phase chromatographiert man den öligen Rückstand über eine Kieselgelsäule mit Dichlormethan/Methanol (95:5) als Elutionsmittel. Die Produktfraktion wird eingeengt, der Rückstand in Methanol gelöst und die Lösung mit etherischer Salzsäure versetzt. Nach erneutem Einengen nimmt man den fest aufgeschäumten gelblichen Rückstand in wenig Dichlormethan auf und fällt die Titelverbindung durch Eintropfen in ca. 1 l Petrolether/Diethylether (2:1) amorph aus.

Man erhält 3,1 g der Titelverbindung als feines Pulver vom Schmp. 124 - 128 °C (langsames Zerfließen).

2. 1,4-Dihydro-2,6-dimethyl-4-(±)-(3-nitrophenyl)-pyridin-3,5-dicarbonsäure-3-methyl-5-{3-[2-(-)-(4-chlorbenzyl)-pyrrolidinyl-1]-propyl}-ester-hydrochlorid

Ein Gemisch aus 1,81 g (±)-1,4-Dihydro-2,6-dimethyl-4-(3-nitrophenyl)-pyridin-3,5-dicarbonsäure-3-methyl-5-(3-brompropyl)-ester, 1,00 g (-)-2-(4-Chlorbenzyl)-pyrrolidin-hydrochlorid und 1,8 g Kaliumcarbonat wird gemäß Verfahrensvariante a) in 20 ml Toluol und 10 ml Wasser unter einer Stickstoffatmosphäre und kräftigem Rühren 20 h unter Rückfluß zum Sieden erhitzt. Nach Abkühlen werden die Phasen getrennt; die organische Phase wird 2 mal mit Wasser gewaschen, über Natriumsulfat getrocknet und eingeengt. Der ölige Rückstand wird über eine Kieselgelsäule chromatographiert (Elutionsmittel: Dichlormethan/Methanol 98:2). Nach Überführung des erhaltenen Amins ins Hydrochlorid wird die Titelverbindung als feines gelbliches Pulver aus Petrolether/Diethylether 3:1 ausgefällt. Schmelzbereich 100 - 116 °C (langsames Zerfließen). Ausbeute: 1,93 g.

3. 1,4-Dihydro-2,6-dimethyl-4-(±)-(3-nitrophenyl)-pyridin-3,5-dicarbonsäure-3-methyl-5-{3-[2-(+)-(4-chlorbenzyl)-pyrrolidinyl-1]-propyl}-ester-hydrochlorid

Analog Beispiel 2 erhält man die Titelverbindung aus 1,81 g (±)-1,4-Dihydro-2,6-dimethyl-4-(3-nitrophenyl)-pyridin-3,5-dicarbonsäure-3-methyl-5-(3-brompropyl)-ester und 1,00 g (+)-2-(4-Chlorbenzyl)-pyrrolidin-hydrochlorid als feines gelbliches Pulver vom Schmelzbereich 125 - 140 °C (langsames Zerfließen). Ausbeute: 1,81 g.

4. 1,4-Dihydro-2,6-dimethyl-4-(+)-(3-nitrophenyl)-pyridin-3,5-dicarbonsäure-3-methyl-5-{3-[2-(-)-(4-chlorbenzyl)-pyrrolidinyl-1]-propyl}-ester-hydrochlorid

Analog Beispiel 2 erhält man die Titelverbindung aus 1,81 g (+)-1,4-Dihydro-2,6-dimethyl-4-(3-nitrophenyl)-pyridin-3,5-dicarbonsäure-3-methyl-5-(3-brompropyl)-ester und 1,00 g (-)-2-(4-Chlorbenzyl)-pyrrolidin-hydrochlorid als feine Kristallplättchen vom Schmp. 113 -117 °C (langsames Zerfließen, aus 2-Propanol) mit $[\alpha]_D^{22}$ = +45,9° (c = 1, Methanol). Ausbeute: 1,87 g. Die freie Base schmilzt bei 81 - 83 °C (aus

Dichlormethan/Diethylether), $[\alpha]_D^{22}$ = +54,7° (c = 1, Methanol); das Fumarat schmilzt bei 105 - 111 °C (langsames Zerfließen, aus Essigsäureethylester), $[\alpha]_D^{22}$ = +40,2° (c = 1, Methanol).

Die Titelverbindung wird ebenfalls erhalten, wenn das in Beispiel 2 hergestellte Diasteromerengemisch der freien Basen mit equimolaren Mengen D-(+)-0,0′-Dibenzoylweinsäure versetzt wird und das aus Aceton/Methanol 1 + 1 zuerst auskristallisierende Salz solange in Aceton/Methanol umkristallisiert wird, bis die spezifische Drehung des D-(+)-0,0′-Dibenzoyltartrates (Hydrat) der Titelverbindung $[\alpha]_D^{22}$ = +77,3° (c = 1, Methanol) beträgt; Schmp.: 153 - 154 °C. Durch Extraktion des Salzes mit Dichlormethan/konz. Ammoniak-Lösung erhält man die freie Base, und aus dieser mit Salzsäure in Diethylether die Titelverbindung.

5. 1,4-Dihydro-2,6-dimethyl-4-(±)-(3-nitrophenyl)-pyridin-3,5-dicarbonsäure-3-methyl-5-{2-[2-(-)-(4-chlorbenzyl)-pyrrolidinyl-1]-ethyl}-ester-hydrochlorid

Analog Beispiel 2 erhält man die Titelverbindung aus 8,80 g (±)-1,4-Dihydro-2,6-dimethyl-4-(3-nitrophenyl)-pyridin-3,5-dicarbonsäure-3-methyl-5-(2-bromethyl)-ester und 4,64 g (-)-2-(4-Chlorbenzyl)-pyrrolidin-hydrochlorid als feines gelbliches Pulver vom Schmelzbereich 123 - 130 °C (langsames Zerfließen). Ausbeute: 3,61 g.

6. 1,4-Dihydro-2,6-dimethyl-4-(±)-(2-chlorphenyl)-pyridin-3,5-dicarbonsäure-3-ethyl-5-{3-[2-(-)-(4-chlorbenzyl)-pyrrolidinyl-1]-propyl}-ester-hydrochlorid

Analog Beispiel 2 erhält man die Titelverbindung, wenn (±)-1,4-Dihydro-2,6-dimethyl-4-(2-chlorphenyl)-pyridin-3,5-dicarbonsäure-3-ethyl-5-(3-brompropyl)-ester eingesetzt wird, als feines gelbliches Pulver vom Schmelzbereich 95 - 105 °C (langsames Zerfließen). Ausbeute: 61 % der Theorie.

7. 1,4-Dihydro-2,6-dimethyl-4-(±)-(2-trifluormethylphenyl)-pyridin-3,5-dicarbonsäure-3-methyl-5-{3-[2-(-)-(4-chlorbenzyl)-pyrrolidinyl-1]-propyl}-ester-hydrochlorid

Bei gleicher Arbeitsweise wie in Beispiel 1 beschrieben erhält man die Titelverbindung gemäß Verfahrensvariante c) aus 2-(2-Trifluormethylbenzyliden)-acetessigsäuremethylester und 3-Aminocrotonsäure-{3-[2-(-)-(4-chlorbenzyl)-pyrrolidinyl-1]-propyl}-ester als feines gelbliches Pulver vom Schmelzbereich 77 - 90 °C (Zersetzung). Ausbeute: 41 % der Theorie.

8. 1,4-Dihydro-2,6-dimethyl-4-(±)-(2,3-dichlorphenyl)-pyridin-3,5-dicarbonsäure-3-methyl-5-{3-[2-(-)-(4-chlorbenzyl)-pyrrolidinyl-1]-propyl}-ester-hydrochlorid

Analog Beispiel 7 erhält man die Titelverbindung, wenn 2-(2,3-Dichlorbenzyliden)-acetessigsäuremethylester eingesetzt wird, als feines gelbliches Pulver vom Schmelzbereich 120 - 132 °C (Zersetzung). Ausbeute: 38 % der Theorie.

9. 1,4-Dihydro-2,6-dimethyl-4-(+)-(3-nitrophenyl)-pyridin-3,5-dicarbonsäure-3-methyl-5-{3-[2-(±)-(4-methoxibenzyl)-pyrrolidinyl-1]-propyl}-ester-hydrochlorid

Analog Beispiel 4 erhält man die Titelverbindung, wenn (±)-2-(4-Methoxibenzyl)-pyrrolidin eingesetzt wird, als feines gelbliches Pulver vom Schmelzbereich 138 - 148 °C (Zersetzung). Ausbeute: 69 % der Theorie.

10. 1,4-Dihydro-2,6-dimethyl-4-(+)-(3-nitrophenyl)-pyridin-3,5-dicarbonsäure-3-methyl-5-{3-[2-(±)-(3,4-dimethoxibenzyl)-pyrrolidinyl-1]-propyl}-ester-hydrochlorid

Analog Beispiel 4 erhält man die Titelverbindung, wenn (±)-2-(3,4-Dimethoxibenzyl)-pyrrolidin eingesetzt wird, als feines gelbliches Pulver vom Schmelzbereich 115 - 126 °C (langsames Zerfließen). Ausbeute: 56

% der Theorie.

11. 1,4-Dihydro-2,6-dimethyl-4-(+)-(3-nitrophenyl)-pyridin-3,5-dicarbonsäure-3-methyl-5-{3-[2-(±)-(4-nitrobenzyl)-pyrrolidinyl-1]-propyl}-ester-hydrochlorid

Analog Beispiel 4 erhält man die Titelverbindung, wenn (±)-2-(4-Nitrobenzyl)-pyrrolidin eingesetzt wird, als feines gelbliches Pulver vom Schmelzbereich 111 - 125 °C (langsames Zerfließen). Ausbeute: 48 % der Theorie.

## Ausgangsverbindungen

A. (±)-2-Acetyl-3-(3-nitrophenyl)-acrylsäure-{3-[2-(4-chlorbenzyl)-pyrrolidinyl-1]-propyl}-ester

a) 14,45 g (±)-Acetessigsäure-{3-[2-(4-chlorbenzyl)-pyrrolidinyl-1]-propyl}-ester, 6,46 g 3-Nitrobenzaldehyd, 3.0 ml Essigsäure und 0,5 ml Piperidin werden in 70 ml Benzol am Wasserabscheider zum Sieden erhitzt. Nach Abscheiden von 0.8 ml Wasser wird die abgekühlte Lösung mit gesättigter Natriumhydrogencarbonatlösung und anschließend mit Wasser gewaschen. Nach dem Trocknen der organischen Phase mit Natriumsulfat engt man die klare Lösung im Hochvakuum bis zur Gewichtskonstanz ein. Die als zähes gebliches Öl zurückbleibende Titelverbindung wird direkt ohne weitere Reinigung zur Kondensation eingesetzt. Ausbeute: 21 g Rohprodukt.

b) 10,72 g (±)-N-(3-Hydroxypropyl)-2-(4-chlorbenzyl)-pyrrolidin werden in 100 ml abs. Toluol gelöst und unter Rühren mit 8,4 ml einer 50 %igen Diketenlösung in Aceton versetzt. Nach mehrtägigem Stehen bei Raumtemperatur (DC-Kontrolle) wird der Ansatz eingeengt und der Rückstand im Hochvakuum getrocknet. Der als hellgelbes, zähes Öl verbleibende (±)-Acetessigsäure-{3-[2-(4-chlorbenzyl)-pyrrolidinyl-1]-propyl}-ester wird ohne weitere Reinigung in Stufe a eingesetzt. Ausbeute: 14,5 g.

c) 10,34 g (±)-2-(4-Chlorbenzyl)-pyrrolidin-hydrochlorid, 6,9 g 3-Brompropanol, 30 g pulverisiertes Kaliumcarbonat und 1 g Kaliumiodid werden in 100 ml eines 1:1 Gemisches aus Dioxan und 1-Butanol 48 h unter Rückfluß und kräftigem Rühren zum Sieden erhitzt. Nach dem Abkühlen wird filtriert, das Filtrat eingeengt. Der ölige Rückstand wird in Essigsäureethylester aufgenommen und die Lösung nochmals filtriert. Nach dem Einengen des Filtrats zur Trockne erhält man (±)-N-(3-Hydroxypropyl)-2-(4-chlorbenzyl)-pyrrolidin als zähviskoses Öl, das ohne weitere Reinigung, wie unter b beschrieben, direkt weiter umgesetzt wird. Ausbeute: 12 g.

B. (±)-1,4-Dihydro-2,6-dimethyl-4-(3-nitrophenyl)-pyridin-3,5-dicarbonsäure-3-methyl-5-(3-brompropyl}-ester

a) 100 g Acetessigsäure-3-brompropyl-ester und 67,7 g 3-Nitrobenzaldehyd werden in 450 ml Benzol gelöst, 22 ml Essigsäure und 2 ml Piperidin zugegeben und dann das Gemisch so lange am Wasserabscheider unter Rückfluß zum Sieden erhitzt, bis etwa 9,5 ml Wassr abgeschieden sind. Nach Abkühlen wird im Vakuum zur Trockne eingeengt, der ölige Rückstand in 500 ml Isopropanol aufgenommen und dann 51,6 g 3-Aminocrotonsäuremethylest3er zugegeben. Unter Rühren wird das Gemisch 4 h auf ca. 50 - 55 °C erwärmt, mit wenigen Kristallen der Titelverbindung angeimpft und dann sehr langsam abgekühlt. Schließlich wird über Nacht bei Raumtemperatur gerührt, dann wird abgesaugt, mit Isopropanol gewaschen und getrocknet. Das Produkt (146,5 g) wird aus der fünffachen Menge Isopropanol (W/V) umkristallisiert und man erhält 132 g der Titelverbindung vom Schmp. 142 - 146 °C. - Auf analoge Weise erhält man (±)-1,4-Dihydro-2,6-dimethyl-4-(2-chlorphenyl)-pyridin-3,5-dicarbonsäure-3-ethyl-5-(3-brompropyl)-ester, wenn anstelle von 3-Nitrobenzaldehyd 2-Chlorbenzaldehyd eingesetzt wird.

b) Ein Gemisch aus 210 g 3-Brom-1-propanol und 1250 ml wasserfreiem Dichlormethan wird mit 1 g 4-Dimethylaminopyridin versetzt. Dann werden unter kräftigem Rühren 300 ml einer 50 %igen Lösung von Diketen in Aceton so zugetropft, daß das Lösungsmittel mäßig siedet. Es wird bei Raumtemperatur stehengelassen, dann einmal mit verdünnter Salzsäure ausgeschüttelt, getrocknet und eingeengt. Der Rückstand wird bei 0,03 mbar destilliert. Man erhält 248 g Acetessigsäure-3-brompropylester vom Kp. 90 °C/0,03 mbar.

C. (+)- und (-)-2-(4-Chlorbenzyl)-pyrrolidin-hydrochlorid

97,8 g (±)-2-(4-Chlorbenzyl)-pyrrolidin werden in 200 ml Aceton gelöst. Die klare Lösung tropft man in 20 Min. zur siedenden Lösung von 118,16 g L-(-)-0,0′-Dibenzoylweinsäure-hydrat in 500 ml Aceton zu. Nach Zutropfen von 2/3 der Acetonlösung beginnt das Ausfallen erster Kristalle. Nach vollständigem Zutropfen wird die erhaltene Kristallsuspension noch weitere 2 h unter Rückfluß zum Sieden erhitzt, danach läßt man die Suspension unter gutem Rühren auf Raumtemperatur abkühlen. Man erhält ein erstes Kristallisat mit $[\alpha]_D^{22}$ = -90,7° (c = 1, Methanol); Ausbeute: 90,1 g. Nach erneutem Umkristallisieren in 600 ml Methanol und unter Zusatz von 100 ml Diisopropylether beim Abkühlen erhält man ein zweites Kristallisat mit $[\alpha]_D^{22}$ = -92,3° (c = 1, Methanol); Ausbeute: 73 g. Aus den gesammelten Mutterlaugen erhält man nach zweimaligem Umkristallisieren in Methanol und unter Zusatz von 20 % Diisopropylether beim Abkühlen ein drittes Kristallisat mit $[\alpha]_D^{22}$ = -92,0° (c = 1, Methanol); Ausbeute: 23 g. Zweites und drittes Kirstallisat (96 g) rührt man zusammen 30 Min. in 870 ml 1 N Natronlauge bis zur vollständigen Auflösung der Kristalle. Die erhaltene Lösung extrahiert man 3 mal mit je 800 ml Dichlormethan, vereinigt die organischen Phasen und wäscht diese noch 2 mal mit je 500 ml Wasser. Nach dem Trocknen der organischen Phase über Natriumsulfat versetzt man mit etherischer Salzsäure, engt die Lösung ein und kristallisiert den erhaltenen festen Rückstand in Methanol/Diisopropylether um. Man erhält 38 g (-)-2-(4-Chlorbenzylpyrrolidin)-hydrochlorid als feine farblose Nadeln vom Schmp. 222 - 223° C, $[\alpha]_D^{22}$ = -31,7° (c = 1, Methanol).

Analog erhält man (+)-2-(4-Chlorbenzylpyrrolidin)-hydrochlorid durch Umkristallisation des Racemates mit D-(+)-0,0′-Dibenzoylweinsäure-hydrat und Aufarbeitung wie oben beschrieben. Schmp.: 220 - 221° C, $[\alpha]_D^{22}$ = + 31,4° (c = 1, Methanol).

D. (+)-1,4-Dihydro-2,6-dimethyl-4-(3-nitrophenyl)-pyridin-3,5-dicarbonsäure-3-methyl-5-(3-brompropyl)-ester

36 g (-)-1-Ethoxymethyl-1,4-dihydro-5-methoxycarbonyl-2,6-dimethyl-4-(3-nitrophenyl)-pyridin-3-carbonsäure/Cinchonidinsalz [Chem. Pharm. Bull. 28(9) 2809-2812 (1980)] werden in 250 ml Chloroform gelöst, mit 260 ml 0,2 N Salzsäurelösung versetzt und kräftig gerührt. Durch Zugabe von 2N Salzsäurelösung wird pH 2 eingestellt, dann werden die Phasen getrennt. Die organische Phase wird insgesamt viermal mit Salzsäurelösung von pH 2 und dann mit Wasser gewaschen, über Natriumsulfat getrocknet und eingeengt. Der ölige Rückstand wird in 200 ml Aceton gelöst. Anschließend werden 16 g feingepulvertes Kaliumcarbonat, 80 ml 1,3-Dibrompropan und 0,5 g [18]-Krone-6 zugegeben. Das Gemisch wird 16 h kräftig bei Raumtemperatur gerührt, dann wird abgesaugt und der Filterkuchen mit Aceton gewaschen. Das Aceton wird am Rotationsverdampfer bei schwachem Vakuum agbezogen und das überschüssige 1,3-Dibrompropan bei 0,02 mbar abdestilliert. Der ölige Rückstand wird unter Eiskühlung mit 160 ml konzentrierter Ameisensäure übergossen; dann wird bei Raumtemperatur so lange gerührt, bis eine klare Lösung entstanden ist (ca. 15 Min.). Die Ameisensäure wird im Vakuum abdestilliert. Nach zweimaliger Zugabe und Abdestillieren von je 50 ml Toluol wird der Rückstand in Dichlormethan gelöst. Die Lösung wird mit Natriumhydrogencarbonat-Lösung ausgerührt (pH 8,5). Die organische Phase wird über Natriumsulfat getrocknet und eingeengt. Der ölige Rückstand wird dann aus Methanol in der Kälte kristallisiert. Man erhält 18,9 g der Titelverbindung vom Schmp.: 112-114° C und $[\alpha]_D^{22}$ = + 13,8° (c = 1, Methanol).

E. (±)-1,4-Dihydro-2,6-dimethyl-4-(3-nitrophenyl)-pyridin-3,5-dicarbonsäure-3-methylester-5-(2-bromethyl)-ester

a) 494 g 2-(3-Nitrobenzyliden)-acetessigsäure-(2-bromethyl)-ester und 173,5 g 3-Aminocrotonsäuremethylester werden unter Rühren und unter einer Stickstoffatmosphäre in 1750 ml Isopropanol bei 75-80° C gelöst. Das Gemisch wird danach sehr langsam im Heizbad abgekühlt. Nach Animpfen mit der Titelverbindung wird 16 h bei Raumtemperatur gerührt, dann gekühlt, abgesaugt, mit eiskaltem Ether gewaschen und getrocknet. Man erhält 481 g der Titelverbindung vom Schmp. 139 -141° C.

b) 405 g Acetessigsäure-2-bromethylester werden in 1500 ml Isopropanol gelöst. Unter Rühren werden 293 g 3-Nitrobenzaldehyd, 4,6 g Essigsäure und 6,8 g Piperidin zugegeben. Das Gemisch wird bei 40° C so lange gerührt bis eine klare Lösung entstanden ist. Man läßt langsam abkühlen, impft mit einigen Kristallen des Endproduktes an und rührt bei Raumtemperatur 48 h lang, dann wird gekühlt, abgesaugt, mit kaltem Isopropanol gewaschen und im Vakuum bei 50° C getrocknet. Man erhält 495 g 2-(3-Nitrobenzyliden)-acetessigsäure-(2-bromethyl)-ester vom Schmp. 94,5 - 95,5° C.

c) 250 g 2-Bromethanol werden in 1,3 l Dichlormethan gelöst und mit 1,2 g 4-Dimethylaminopyridin versetzt. Unter kräftigem Rühren werden 400 ml einer 50%igen Diketenlösung in Aceton so zugetropft, daß das Lösungsmittel mäßig siedet. Nach dem Zutropfen wird noch 1 h bei Siedetempratur gerührt und dann über Nacht stehengelassen. Nach Abdampfen des Lösungsmittels am Rotationsverdampfer im Vakuum wird der Rückstand destilliert. Man erhält 402 g Acetessigsäure-2-bromethylester mit Kp. 80 - 83 °C/0,04 mbar.

F. 3-Aminocrotonsäure-{3-[2-(-)-(4-chlorbenzyl)-pyrrolidinyl-1]-propyl}-ester

In eine Lösung von Acetessigsäure-{3-[2-(-)-(4-chlorbenzyl)-pyrrolidinyl-1]-propyl}-ester in 2-Propanol wird unter Eiskühlung und Rühren bis zur Sättigung Ammoniakgas eingeleitet. Nach Stehenlassen über Nacht bei 0 °C engt man den Ansatz im Wasserstrahlvakuum bei maximal 70 °C Badtemperatur bis zur Gewichtskonstanz ein. Das erhaltene gebliche Öl wird ohne weitere Reinigung direkt weiter umgesetzt.

## Gewerbliche Anwendbarkeit

Die erfindungsgemäßen Verbindungen der Formel I und ihre Salze besitzen wertvolle Eigenschaften, die sie gewerblich verwertbar machen. Sie stellen insbesondere wirksame Vasodilatoren mit coronartherapeutischen Eigenschaften dar. Die pharmakologische Wirksamkeit der erfindungsgemäßen Verbindungen, die gepaart ist mit einer geringen Toxizität, zeigt sich insbesondere in einer rasch eintretenden, starken und optimal anhaltenden Blutdrucksenkung nach oraler Applikation. Darüberhinaus besitzen die erfindungsgemäßen Verbindungen hemmende Wirkung auf den Calciumeinstrom sowie fördernde Wirkung auf den Kaliumsstrom von Zellen, glattmuskulär relaxierende und peripher, coronar, cerebral und renal gefäßerweiternde sowie salidiuretische, antithrombotische, antiarteriosklerotische und günstige hämorheologische Eigenschaften.

In ihrer ausgezeichneten Wirksamkeit, die gepaart ist mit einer geringen Toxizität und dem Fehlen wesentlicher Nebenwirkungen, unterscheiden sich die erfindungsgemäßen Verbindungen in überraschender und vorteilhafter Weise von den Verbindungen des Standes der Technik.

Als vorteilhafte Eigenschaften der Verbindungen I sind beispielsweise zu nennen: das Ausmaß der Blutdrucksenkung, das optimale Anhalten der Blutdrucksenkung, die gute Steuerbarkeit der Blutdrucksenkung, die überrashcend geringe Herzfrequenzsteigerung, die ausgezeichnete Bioverfügbarkeit, die große therapeutische Breite, das Fehlen zentraler Nebenwirkungen, das Fehlen kinetischer Interaktionen mit anderen Substanzen, das Ausbleiben einer Toleranzentwicklung, die ausgewogenen physikalischen Eigenschaften und die große Stabilität.

Die ausgezeichnete Wirksamkeit der erfindungsgemäßen Verbindungen der Formel I und ihrer Salze gestattet ihren Einsatz in der Humanmedizin, wobei als Indikation insbesondere primäre (essentielle) und sekundäre, arterielle und pulmonale Hypertonien aller Schweregrade, koronare Herzkrankheiten (Koronarinsuffizienz, Angina Pectoris, Myocardinfarkt etc.), periphere und cerebrale Zirkulationsstörungen (Gehirnschlag, temporäre cerebrale Durchblutungsstörungen, Migräne, Schwindel, renale Arterienverengung etc.), hypertrophe Kardiomyopathie, Herzinsuffizienz, Krankheiten, die auf einer erhöhten Wasser- und Natriumretention beruhen und Krankheiten, die auf einem erhöhten Calciumeinstrom beruhen, wie z.B. Spasmen glattmuskulärer Organe (Atemwege, Gastrointestinaltrakt, Urogenitaltrakt etc.) sowie Arrhythmie, Arteriosklerose und Zellschädigungen verschiedener Genese (z.B. Hypoxie) in Betracht kommen.

Ein weiterer Gegenstand der Erfindung ist daher ein Verfahren zur Behandlung von Säugetieren, insbesondere Menschen, die an einer der obengenannten Krankheiten erkrankt sind. Das Verfahren ist dadurch gekennzeichnet, daß man dem erkrankten Individuum eine therapeutisch wirksame und pharmakologisch verträgliche Menge einer oder mehrerer Verbindungen der Formel I verabreicht.

Gegenstand der Erfindung sind außerdem die Verbindungen der Formel I zur Anwendung bei der Behandlung der genannten Krankheiten.

Ebenso umfaßt die Erfindung die Verwendng von Verbindungen der Formel I bei der Herstellung von Arzneimitteln, die zur Bekämpfung der genannten Krankheiten eingesetzt werden.

Ein weiterer Gegenstand der Erfindung sind Arzneimittel, die eine oder mehrere Verbindungen der allgemeinen Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form

von Tabletten, Dragees, Kapseln, Suppositorien, Pflastern (z.B. als TTS), Emulsionen, Suspensionen, Aerosolen, Sprays, Salben, Cremes, Gelen oder Lösungen eingesetzt, wobei der Wirkstoffgehalt vorteilhafterweise zwischen 0,1 udn 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, rektal, per inhalationem oder parenteral (insbesondere perlingual, intravenös oder percutan) appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 10, vorzugsweise 0,05 bis 5 mg/kg Körpergewicht, gewünschtenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Bei einschleichender Dosierung wird zu Beginn der Behandlung eine geringere Dosis verabreicht, dann langsam auf eine höhere Dosis übergegangen. Nach Erreichen des gewünschten Therapieerfolges wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der genannten Krankheiten eingesetzt werden, so können die pharmazeutischen Zubereitungen auch einen oder mehrere andere pharmakologisch aktive Bestandteile anderer Arzneimittelgruppen, wie andere Vasodilatoren, Antihypertensiva, alpha-1-Rezeptorenblocker, alpha-2-Rezeptorstimulatoren, beta-1-Rezeptorenblocker, beta-2-Rezeptorstimulatoren, ACE-Hemmstoffe, Nitroverbindungen, Cardiotonika, Diuretika, Saluretika, Alkaloide, Analgetika, Lipidsenker, Antikoagulantien, Anticholinergika, Methylxanthine, Antiarrhythmika, Antihistaminika, Dopaminstimulatoren, Serotonin-Rezeptorenblocker etc., wie Nifedipin, Dihydralazin, Prazosin, Clonidin, Atenolol, Labetalol, Fenoterol, Captopril, Isosorbiddinitrat, Digoxin, Milrinon, Mefrusid, Clopamid, Spironolacton, Chlorthalidon, Furosemid, Polythiazid, Hydrochlorothiazid, Reserpin Dihydroergocristin, Rescinnamin, Rauwolfia-Gesamtalkaloide, Acetylsalicylsäure, Bezafibrat, Warfarin, Atropin, Theophyllin, Lidocain, Astemizol, Bromocryptin, Ketanserin etc. enthalten.

## Pharmakologie

Die antihypertensive Wirksamkeit der erfindungsgemäßen Verbindungen kann am Modell der spontan hypertonen Ratte nachgewiesen werden.

Zur Bestimmung der antihypertensiven Wirkung werden die unten aufgeführten Verbindungen in den angegebenen Dosen an vier aufeinander folgenden Tagen an je 6 männlichen Ratten (Stamm SHR/N/Ibm/Bm, 250-350 g) mit genetisch bedingtem Hochdruck (systolischer Blutdruck > 180 mmHg) täglich einmal mittels Schlundsonde verabfolgt. Die Messung des Blutdrucks erfolgt jeweils 6 und gegebenenfalls 2 oder 24 Stunden nach Substanzgabe.

Die Blutdruckmessung wird in einer Wärmekammer bei 36 °C vorgenommen, um eine bessere Durchblutung der Schwanzarterie zu erreichen. Hierzu werden die Tiere in perforierte Lochblechkäfige verbracht und 20 - 40 Min. nach Beginn der Aufwärmung gemessen. Zur Messung des systolischen arteriellen Drucks wird eine ringförmige Manschette mit aufblasbarer Gummimembran zur Unterbindung der Durchblutung und ein ringförmiger Piezokristallaufnehmer zur Erfassung der Pulswellen auf den Schwanz aufgeschoben. Nach erfolgter Unterbindung des Blutstroms in der Schwanzarterie wird der Manschettendruck kontinuierlich reduziert. Die Wiederkehr der Pulswellen bei Druckablassen wird automatisch als systolischer Blutdruck erkannt und ausgedruckt (Bühler, R. et al.,: Microprocessor-based automation of blood pressure measurement in the conscious rat. Proceedings of the 4th international symposium on rats with spontaneous hypertension and related studies, Rascher, R. et al. (Eds.), Schattauer Verlag, Stuttgart, New York, 1982, S. 410-413). Pulssignale und Druckverlauf werden zur Auswertung graphisch aufgezeichnet.

Zur Gewöhnung an den Meßvorgang werden die Tiere vor Substanzprüfung 14 Tage trainiert. In der zweiten Trainingswoche werden Blutdruck-Vorwerte erhoben. Tiergruppen, die Substanz erhalten, werden gegen eine Kontrollgruppe geprüft.

In der anschließenden Tabelle werden die untersuchten Verbindungen durch laufende Nummern gekennzeichnet, die mit den jeweiligen Beispielnummern übereinstimmen.

Tabelle I gibt für die Vertreter der erfindungsgemäßen Verbindungen die prozentuale Senkung des Blutdrucks (BP) nach oraler Verabreichung bei der Ratte wieder.

Tabelle I

| %-Änderungen (BP) an genetisch hypertonen Ratten nach täglich einmaliger p.o.-Applikation an vier aufeinanderfolgenden Tagen (N = 6/Dosis). | | | | |
|---|---|---|---|---|
| Beisp. Nr. | Dosis µmol/kg | BP (% Änderung vs. Kontrolle), Mittelwert für Meßzeitpunkte: | | |
| | | Stunden nach Gabe (Tage) | | |
| | | 2h (1. + 4.Tag) | 6h (1.-4.Tag) | 24h (1. + 3.Tag) |
| 1 | 25 | -55,5 | -43,5 | - 3 |
| 2 | 25 | -47 | -34 | -13,5 |
| 4 | 10 | -48 | -33 | - 8 |
| 8 | 10 | -39 | -25,5 | + 2 |
| 11 | 25 | -36,5 | -25,5 | - 8 |

## Ansprüche

1. Pyrrolidine der Formel I

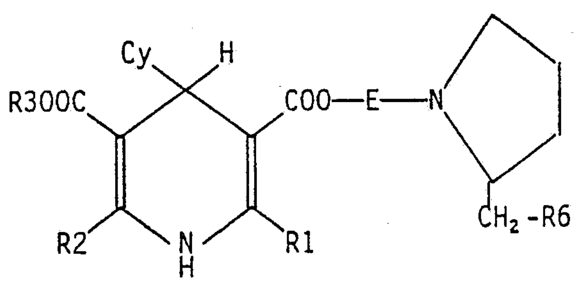

(I)

worin Cy einen Cyclus der Formel

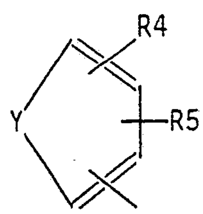

darstellt, in dem Y Sauerstoff (O), Schwefel (S), Vinylen (-CH = CH-), Azomethin (-CH = N-) oder eine Gruppe der Formel

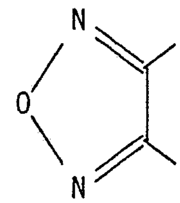

oder

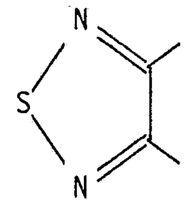

bedeutet,
R1 1-6C-Alkyl oder 3-7C-Alkoxyalkyl bedeutet,
R2 1-6C-Alkyl oder 3-7C-Alkoxyalkyl bedeutet,
R3 Wasserstoff, 1-6C-Alkyl oder 3-7C-Alkoxyalkyl bedeutet,
R4 und R5 gleich oder verschieden sind und Wasserstoff, Hydroxy, Halogen, Nitro, Cyano, Trifluormethyl, 1-4C-Alkyl, 1-4C-Alkoxy, ganz oder teilweise durch Fluor substituiertes 1-4C-Alkoxy, 1-4C-Alkoxycarbonyl, 2-5C-Acyl, Amino oder Mono- oder Di-1-4C-alkylamino bedeuten,
R6 Phenyl oder substituiertes Phenyl mit einem, zwei oder drei gleichen oder verschiedenen Substituenten aus der Gruppe Halogen, Hydroxy, Nitro, Cyano, Trifluormethyl, 1-4C-Alkyl, 1-4C-Alkoxy, 1-4C-Alkoxycarbonyl, 2-5C-Acyl, Amino und Mono- oder Di-1-4C-alkylamino bedeutet und
E 2-5C-Alkylen bedeutet,
und die Salze dieser Verbindungen.

2. Verbindungen der Formel I nach Anspruch 1, worin
Cy Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dicylorphenyl, 2-Fluorphenyl, 3-Fluorphenyl, 2-Trifluormethylphenyl, 3-Trifluormethylphenyl oder Benzodiazolyl bedeutet,
R1 Methyl, Ethyl oder Methoxyethyl bedeutet,
R2 Methyl, Ethyl oder Methoxyethyl bedeutet,
R3 Methyl, Ethyl oder Methoxyethyl bedeutet,
R6 Phenyl, 2-Chlorphenyl, 3-Chlorphenyl, 4-Chlorphenyl, 2,3-Dichlorphenyl, 3,4-Dichlorphenyl, -2,4-Dichlorphenyl, 4-Bromphenyl, 4-Fluorphenyl, 3-Trifluormethylphenyl, 2-Methoxyphenyl, 3-Methoxyphenyl, 4-Methoxyphenyl, 3,4-Dimethoxyphenyl, 3,4,5-Trimethoxyphenyl, 2-Methylphenyl, 3-Methylphenyl, 3-Chlor-4-methylphenyl, 3,4-Dimethylphenyl, 4-Aminophenyl, 2,4-Diaminophenyl, 4-Nitrophenyl, 3-Hydroxyphenyl, 4-Hydroxyphenyl, 3,4-Dihydroxyphenyl oder 3,4,5-Trihydroxyphenyl bedeutet und
E Ethylen ($-CH_2CH_2-$), Trimethylen ($-CH_2-CH_2-CH_2-$), Tetramethylen ($-CH_2-CH_2-CH_2-CH_2-$) oder Pentamethylen ($-CH_2-CH_2-CH_2-CH_2-CH_2-$) bedeutet,
und die Salze dieser Verbindungen.

3. Verbindungen der Formel I nach Anspruch 1, worin
Cy 3-Nitrophenyl, 2-Chlorphenyl, 2,3-Dichlorphenyl oder 2-Trifluormethylphenyl bedeutet,
R1 Methyl oder Ethyl bedeutet,
R2 Methyl oder Ethyl bedeutet,
R3 Methyl oder Ethyl bedeutet,
R6 4-Chlorphenyl, 4-Methoxyphenyl, 3,4-Dimethoxyphenyl oder 4-Nitrophenyl bedeutet und
E Ethylen ($-CH_2CH_2-$) oder Trimethylen ($-CH_2-CH_2-CH_2-$) bedeutet,
und die Salze dieser Verbindungen.

4. Verbindungen der Formel I nach Anspruch 1, worin
Cy 3-Nitrophenyl oder 2,3-Dichlorphenyl bedeutet,
R1 Methyl oder Ethyl bedeutet,
R2 Methyl oder Ethyl bedeutet,
R3 Methyl oder Ethyl bedeutet,
R6 4-Chlorphenyl oder 4-Nitrophenyl und
E Ethylen ($-CH_2CH_2-$) oder Trimethylen ($-CH_2-CH_2-CH_2-$) bedeutet,
und ihre Salze.

5. Verbindungen der Formel I nach Anspruch 1, worin
Cy 3-Nitrophenyl bedeutet,
R1 Methyl bedeutet,
R2 Methyl bedeutet,
R3 Methyl oder Ethyl bedeutet,

R6 4-Chlorphenyl und
E Ethylen ($-CH_2CH_2-$) oder Trimethylen ($-CH_2-CH_2-CH_2-$) bedeutet,
und ihre Salze.

6. 1,4-Dihydro-2,6-dimethyl-4-(+)-(3-nitrophenyl)-pyridin-3,5-dicarbonsäure-3-methyl-5{3-[2-(-)-(4-chlorbenzyl)-pyrrolidinyl-1]propyl}-ester und seine Salze.

7. Verfahren zur Herstellung von Verbindungen der Formel I nach Anspruch 1 und ihren Salzen, dadurch gekennzeichnet, daß man
a) 1,4-Dihydropyridinderivate der Formel II

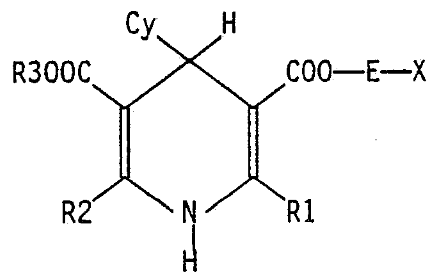

(II),

mit Aminen der Formel III

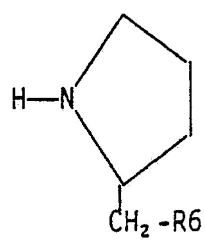

(III),

oder
b)Enamine der Formel IV

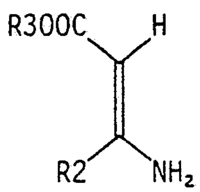

(IV),

mit Benzylidencarbonsäurederivaten der Formel V

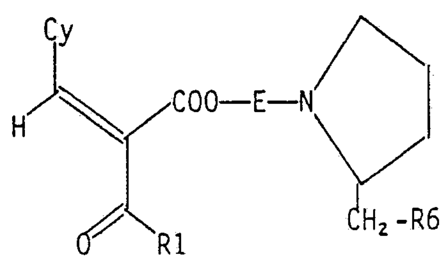

(V),

oder
c) Enaminderivate der Formel VI

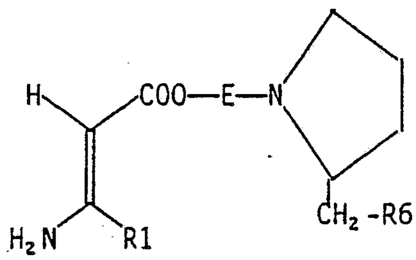

(VI),

mit Zimtsäurederivaten der Formel VII

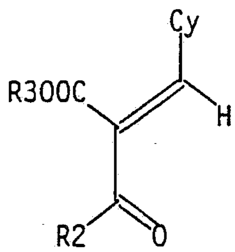

(VII),

als solche(n) oder in Form ihrer Salze umsetzt und gewünschtenfalls anschliessend erhaltene Salze in die freien Basen oder erhaltene Basen in die Salze überführt, wobei Cy, R1, R2, R3, R4, R5, R6 und E die in Anspruch 1 angegebenen Bedeutungen haben und X eine Fluchtgruppe darstellt.

8. Arzneimittel enthaltend eine oder mehrere Verbindungen nach einem oder mehreren der Ansprüche 1 bis 6 und/oder ihre pharmakologisch verträglichen Salze.

9. Verbindungen nach einem oder mehreren der Ansprüche 1 bis 6 und ihre pharmakologisch verträglichen Salze zur Anwendung bei der Behandlung und/oder Prophylaxe von Hypertonie, koronaren Herzkrankheiten, peripheren und cerebralen Zirkulationsströrungen und/oder Krankheiten, die auf einer erhöhten Wasser- oder Natriumretention beruhen.

Europäisches Patentamt

# EUROPÄISCHER RECHERCHENBERICHT

Nummer der Anmeldung

EP 88 11 7660

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | WO-A-8 603 748 (BYK LOMBERG) * Insgesamt * | 1-9 | C 07 D 24/90<br>C 07 D 401/12<br>C 07 D 401/14<br>C 07 D 413/14<br>A 61 K 31/445 |
| X | WO-A-8 402 702 (TAKEDA) * Zusammenfassung * | 1-9 | |
| X | WO-A-8 303 249 (YOSHITOMI PHARM.) * Zusammenfassung * | 1-9 | |
| A | GB-A-2 142 021 (SANDOZ LTD) * Insgesamt * | 1-9 | |
| A | EP-A-0 176 956 (BYK LOMBERG) * Insgesamt * | 1-9 | |
| | | | **RECHERCHIERTE SACHGEBIETE (Int. Cl.4)** |
| | | | C 07 D 211/00<br>C 07 D 401/00<br>C 07 D 413/00 |

Der vorliegende Recherchenbericht wurde für alle Patentansprüche erstellt

| Recherchenort | Abschlußdatum der Recherche | Prüfer |
|---|---|---|
| DEN HAAG | 24-11-1988 | MAISONNEUVE J.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)